# EUROPÄISCHE PATENTSCHRIFT

(11) **EP 1 169 281 B1**
(45) Veröffentlichungstag und Bekanntmachung des Hinweises auf die Patenterteilung: **18.02.2004**
(21) Anmeldenummer: 00922539.2
(22) Anmeldetag: 23.03.2000
(51) Int. Cl.: C07B 41/14, C07C 409/04

(54) **SINGLET SAUERSTOFF OXIDATION VON ORGANISCHEN SUBSTRATEN**
SINGLET OXYGEN OXIDATION OF ORGANIC SUBSTRATES
OXYDATION D'OXYGENE SINGULET DE SUBSTRATS ORGANIQUES

(30) Priorität: 13.04.1999 AT 64799
(43) Veröffentlichungstag der Anmeldung: 09.01.2002
(73) Patentinhaber: DSM Fine Chemicals Austria Nfg GmbH & Co KG, 4021 Linz (AT)
(72) Erfinder: AUBRY, Jean-Marie, F-62590 Oignies (FR); RATAJ-NARDELLO, Véronique, F-59710 Pont-a-Marcq (FR); ALSTERS, Paul, NL-6224 KZ Maastricht (NL)
(74) Vertreter: Lindinger, Ingrid
(86) Internationale Anmeldenummer: PCT/EP2000/002552
(87) Internationale Veröffentlichungsnummer: WO 2000/061524

(56) Entgegenhaltungen:
- D. H. R. BARTON: "Experiments on the synthesis of tetracycline. Part XIII. Oxidation of ring A model phenols to p-hydroxycyclohexadienones" JOURNAL OF THE CHEMICAL SOCIETY, PERKIN TRANSACTIONS 1, 1975, Seiten 1610-1614, XP002145295 LETCHWORTH GB
- F. VAN LAAR: "Heterogeneous molybdate catalysts for the generation of singlet molecular oxygen from H2O2" JOURNAL OF THE CHEMICAL SOCIETY, CHEMICAL COMMUNICATIONS, 1998, Seiten 267-268, XP002145394 LETCHWORTH GB
- J-M AUBRY: "Preparative oxidation of organic compounds in microemulsions with singlet oxygen generated chemcally by the sodium molybdate/hydrogen peroxide system" JOURNAL OF THE AMERICAN CHEMICAL SOCIETY, Bd. 119, Nr. 23, 11. Juni 1997 (1997-06-11), Seiten 5286-5294, XP002145395 DC US in der Anmeldung erwähnt
- J. M. AUBRY: "Chemical sources of singlet oxygen. 3. Peroxidation of water-soluble singlet oxygen carriers with the hydrogen peroxide-molybdate system" JOURNAL OF ORGANIC CHEMISTRY, Bd. 54, Nr. 3, 1989, Seiten 726-728, XP002145396 EASTON US in der Anmeldung erwähnt

## Beschreibung

Die einzige Singlet Sauerstoff Oxidation (¹O₂-Ox), die zur Zeit industriell durchgeführt wird, ist die photochemische ¹O₂-Ox, bei welcher der ¹O₂ auf photochemischen Weg generiert wird. Der Nachteil dieses Verfahrens ist durch die hohen Kosten der benötigten photochemischen Einrichtungen, sowie durch eine beschränkte Lebensdauer gegeben. Die benötigten Lampen degenerieren durch Verschmutzung der Glasoberfläche relativ rasch während der Oxidation. Außerdem eignet sich dieses Verfahren nicht für gefärbte Substrate. Das Verfahren ist eigentlich nur für Feinchemikalien geeignet, die im kleineren Maßstab hergestellt werden. (La Chimica e l'Industria, 1982, Vol. 64, Seite 156) Aus diesem Grund wurde versucht andere Verfahrensvarianten für die ¹O₂-Ox zu finden, die für die ¹O₂-Ox von nicht-wasserlöslichen, hydrophoben organischen Substraten geeignet sind.

In J. Am. Chem. Soc., 1968, **90**, 975 wird beispielsweise die klassische "dark" ¹O₂-Ox beschrieben, bei welcher ¹O₂ nicht photochemisch sondern chemisch generiert wird. Dabei werden hydrophobe Substrate mittels eines Hypochlorit/H₂O₂ - Systems in einem Lösungsmittelgemisch aus Wasser und organischem Lösungsmittel oxidiert. Dieses Verfahren hat jedoch lediglich einige synthetische Anwendungen gefunden, da viele Substrate in dem benötigtem Medium nur schwer löslich sind. Die Einsatzmöglichkeit ist außerdem aufgrund von Nebenreaktionen zwischen Hypochlorit und Substrat oder Lösungsmittel ziemlich eingeschränkt. Außerdem wird in der Gasphase ein großer Teil des ¹O₂ deaktiviert. Weiters ist dieses Verfahren nicht für den industriellen Maßstab geeignet, da es im organischen Medium zur Anlagerung des Hypochlorits an H₂O₂ kommt und ein großer Überschuss an H₂O₂ zur Unterdrückung der Nebenreaktion von Substrat mit Hypochlorit benötigt wird. Ein zusätzlicher Nachteil ergibt sich durch das Anfallen stöchiometrischer Salzmengen.

Eine Variante der "dark" ¹O₂-Ox, die nicht auf Hypochlorit basiert und somit obige Nachteile zum Teil vermeiden soll, ist beispielsweise aus J. Org. Chem., 1989, **54**, 726 oder J. Mol. Cat., 1997, **117,** 439 bekannt, wonach einige wasserlösliche organische Substrate mit H₂O₂ und einem Molybdatkatalysator in Wasser als Lösungsmittel oxidiert werden. Gemäß Membrane Lipid Oxid. Vol. II, 1991, 65 ist die ¹O₂-Ox von wasserunlöslichen, organischen Substraten mit dem Molybdat/ H₂O₂ -System schwierig, da angenommen wurde, dass keines der üblichen Lösungsmittel geeignet ist, die von Molybdat katalysierte Disproportionierung von H₂O₂ in Wasser und ¹O₂ aufrecht zu erhalten. Wie in Membrane Lipid Oxid. Vol. II, 1991, 65 beschrieben ist, können wasserunlösliche Substrate, wie etwa α-Terpinen oder β-Citronellol mit dem Molybdat/H₂O₂ -System in einem MeOH/Wasser-Gemisch (70/30) mit nur relativ geringen Ausbeuten von 70% oxidiert werden. Neben den nur mittelmäßigen Ausbeuten, die unter Verwendung von MeOH/Wasser erhalten werden, ist das sehr geringe Anwendungsspektrum dieser auf wässrigen Lösungsmittelgemischen beruhenden Methode ein zusätzlicher Nachteil, da diese Methode, wie aus J.Am.Chem.Soc., 1997, **119**, 5286 hervor geht, auf etwas hydrophile Substrate oder auf hydrophobe Substrate mit geringem Molekulargewicht eingeschränkt ist.

In J.Am.Chem.Soc., 1997, **119**, 5286 und EP-A-0 288 337 ist ein Verfahren beschrieben, dass die von Molybdat in wässriger Lösung katalysierte Generierung von ¹O₂ aus H₂O₂ ermöglicht und trotzdem für hydrophobe Substrate aus einem breiteren Molekulargewichtsbereich geeignet ist. In diesem Verfahren wird eine Mikroemulsion als Reaktionsmedium verwendet. Die Anwendung im industriellen Maßstab ist jedoch mit Problemen verbunden, da sich die Produktisolierung aus der Mikroemulsion schwierig gestaltet. Weiters handelt es sich um ein relativ teures Verfahren, da ziemlich große Mengen an Tensid relativ zum Substrat eingesetzt werden müssen.

Aus J.C.S. Perkin; 1975, Seite 1610 - 1614 ist bekannt, dass verschiedene Phenole mit einem deutlichen molaren Überschuss an Cer(IV)-Oxid in Bezug auf das Substrat in Gegenwart von H₂O₂ über ¹O₂ oxidiert werden können.

In Chem. Commun., 1998, Seite 267-268 wird hingegen die Verwendung eines speziellen heterogenen Mo-Katalysators beschrieben, wobei darauf hingewiesen wird, dass durch die Verwendung dieses heterogenen Katalysators die bei Verwendung von homogenen Mo-Katalysatoren notwendige Zugabe einer löslichen Base überflüssig macht.

Aufgabe der vorliegenden Erfindung war es demnach, eine verbesserte Methode der "dark" ¹O₂-Ox zu finden, die einfach, kostengünstig und umweltfreundlich im industriellen Maßstab angewendet werden kann und für eine Vielzahl von Substraten geeignet ist.

Unerwarteterweise wurde nun gefunden, dass die "dark" ¹O₂-Ox in äußerst effizienter Weise mit hoher Ausbeute in bestimmten organischen Lösungsmittel als Reaktionsmedium, ohne Zugabe von Wasser als Co-Lösungsmittel und ohne Tensid durchgeführt werden kann.

Gegenstand der vorliegenden Erfindung ist demnach ein Verfahren zur Oxidation von organischen Substraten mittels ¹O₂, das dadurch gekennzeichnet ist, dass hydrophobe, organische Substrate aus der Gruppe Olefine, die 1 bis 6 C=C-Doppelbindungen enthalten; C₆-C₃₀ Phenole, Polyalkylbenzole, Polyalkoxybenzolen; polycyclische Aromaten mit 2 bis 6 aromatischen Ringen; sowie C₄-C₂₀ Heterocyclen mit einem O-, N-oder S-Atom im Ring, die unsubstituiert sein können oder ein- oder mehrfach mit Halogenen, Cyanid, Carbonylgruppen, Hydroxylgruppen, C₁-C₂₀-Alkoxygruppen, C₁-C₂₀-Alkylgruppen, C₆-C₂₀-Arylgruppen, C₂-C₂₀-Alkenylgruppen, C₂-C₂₀-Alkinylgruppen, Carbonsäuregruppen, Estergruppen, Amidgruppen, Aminogruppen, Nitrogruppen, Silylgruppen, Silyloxygruppen, Sulfongruppen, Sulfoxidgruppen oder mit einem oder mehreren NR¹R²-Resten, in denen R₁ oder R₂ gleich oder verschieden sein können und H; C₁-C₂₀-Alkyl; Formyl; C₂-C₂₀-Acyl; C₇-C₂₀-Benzoyl bedeuten, wobei R¹ und R² auch gemeinsam einen Ring bilden können, substituiert sein können, in einem organischem Lösungsmittel aus der Gruppe C₁-C₈-Alkohole, Formamid, N-Methylformamid, Dimethylformamid, Sulfolan und Propylencarbonat in Gegenwart eines homogenen Katalysators basierend auf Molybdän, Wolfram, Scandium, Vanadium, Titan, Zirkon, Praseodym, Neodym, Samarium, Europium, Terbium, Dysprosium, Holmium, Erbium, Ytterbium oder Lutetium in Form von Oxiden, Oxokomplexen, Nitraten, Carboxylaten, Hydroxiden, Carbonaten, Chloriden, Fluoriden, Sulfaten oder Tetrafluorboraten mit 30-70%igem H₂O₂ versetzt werden, worauf anschließend an die katalytische Zersetzung von H₂O₂ zu Wasser und ¹O₂ die Oxidation zu den entsprechenden Oxidationsprodukten erfolgt.

Das erfindungsgemäße Verfahren eignet sich zur Oxidation von hydrophoben, organischen Substraten, die mit ¹O₂ reagieren.
Als Substrat können demnach folgende Verbindungen eingesetzt werden: Olefine, die eine oder mehrere, d.h. bis zu 6, bevorzugt bis zu 4 C=C-Doppelbindungen enthalten; elektronenreiche Aromaten, wie C₆-C₂₀-Phenole, Polyalkylbenzole, Polyalkoxybenzolen; polycyclische Aromaten mit 2 bis 6, bevorzugt bis 4 aromatischen Ringen; sowie Heterocyclen mit einem O-, N- oder S-Atom im Ring, wie beispielsweise C₄-C₂₀-Furane, C₄-C₂₀-Pyrrole, C₄-C₂₀-Thiophene. Die Substrate können dabei einen oder mehrere Substituenten, wie Halogen (F, Cl, Br, J), Cyanid, Carbonylgruppen, Hydroxylgruppen, C₁-C₂₀-Alkoxygruppen, C₁-C₂₀-Alkylgruppen, C₆-C₂₀-Arylgruppen, C₂-C₂₀-Alkenylgruppen, C₂-C₂₀-Alkinylgruppen, Carbonsäuregruppen, Estergruppen, Amidgruppen, Aminogruppen, Nitrogruppen, Silylgruppen, Silyloxygruppen, Sulfongruppen, Sulfoxidgruppen, aufweisen. Weiters können die Substrate substituiert sein mit einem oder mehreren NR¹R²-Resten, in denen R₁ oder R₂ gleich oder verschieden sein können und H; C₁-C₂₀-Alkyl; Formyl; C₂-C₂₀-Acyl; C₇-C₂₀-Benzoyl bedeuten, wobei R¹ und R² auch gemeinsam einen Ring bilden können, wie z.B. in einer Phthalimidogruppe.
Beispiele für geeignete Substrate sind: 2-Buten; Isobuten; 2-Methyl-1-buten; 2-Hexen; 1,3-Butadien; 2,3-Dimethylbuten; D^{9,10}-Octalin, 2-Phthalimido-4-Methyl-3-penten; 2,3,-Dimethyl-1,3-Butadien; 2,4-Hexadien; 2-Chlor-4-methyl-3-penten; 2-Brom-4-methyl-3-penten; 1-Trimethylsilylcyclohexen; 2,3-Dimethyl-2-butenyl-*para*-tolylsulfon; 2,3-Dimethyl-2-butenyl-*para*-tolylsulfoxid; *N*-Cyclohexenylmorpholin; 2-Methyl-2-norbornen; Terpinolen; α-Pinen; β-Pinen; β-Citronellol; Ocimen; Citronellol; Geraniol; Farnesol; Terpinen; Limonen; *trans*-2,3-Dimethylacrylsäure; α-Terpinen; Isopren; Cyclopentadien; 1,4-Diphenylbutadien; 2-Ethoxybutadien; 1,1'-Dicyclohexenyl; Cholesterol; Ergosterolacetat; 5-Chlor-1,3-cyclohexadien; 3-Methyl-2-buten-1-ol; 3,5,5-Trimethylcyclohex-2-en-1-ol; Phenol, 1,2,4-Trimethoxybenzol, 2,3,6-Trimethylphenol, 2,4,6-Trimethylphenol, 1,4-Dimethylnaphthalen, Furan, Furfurylalcohol, Furfural, 2,5-Dimethylfuran, Isobenzofuran, Dibenzylsulfid, (2-Methyl-5-*tert*-butyl)phenylsulfid u.s.w.

Aus den Substraten wird durch die erfindungsgemäße Oxidation das korrespondierende Oxidationsprodukt erhalten. Aus Alkenen, (polycyclischen) Aromaten oder Heteroaromaten werden insbesondere Hydroperoxide oder Peroxide erhalten, die unter den Reaktionsbedingungen zu Alkoholen, Epoxiden, Acetalen oder Carbonylverbindungen, wie Ketone, Aldehyde, Carbonsäuren oder Ester weiter reagieren können, wenn das Hydroperoxid oder das Peroxid nicht stabil ist.

Die erfindungsgemäße Oxidation erfolgt in einem organischem Lösungsmittel aus der Gruppe C₁-C₈-Alkohole, wie Methanol, Ethanol, Propanol, i-Propanol, Butanol, i-Butanol, Ethylenglykol, Propylenglykol, Formamid, N-Methylformamid, Dimethylformamid, Sulfolan, Propylencarbonat.
Bevorzugt werden Methanol, Ethanol, Propanol, i-Propanol, Ethylenglykol, Propylenglykol, Formamid, N-Methylformamid oder Dimethylformamid, besonders bevorzugt Methanol, Ethanol, Ethylenglykol, Propylenglykol, Formamid oder Dimethylformamid als Lösungsmittel eingesetzt.

Dem Lösungsmittel-Substrat-Gemisch wird als homogener anorganischer Katalysator ein Katalysatoren basierend auf Molybdän, Wolfram, Scandium, Vanadium, Titan, Zirkon, Praseodym, Neodym, Samarium, Europium, Terbium, Dysprosium, Holmium, Erbium, Ytterbium,und Lutetium zugesetzt.
Der Katalysator kann dabei in für ¹O₂-Oxidationen üblichen Formen, beispielsweise als Oxid, Oxokomplex, Nitrat, Carboxylat, Hydroxid, Carbonat, Chlorid, Fluorid, Sulfat, Tetrafluorborat, u.s.w., vorliegen.
Bevorzugt sind Molybdän-Katalysatoren.

Die Menge an eingesetztem Katalysator hängt vom eingesetzten Substrat ab und liegt zwischen 1 und 50 mol%, bevorzugt zwischen 5 und 25 mol%

Anschließend erfolgt die Zugabe von 30-70%igem, bevorzugt 40-60%igem H₂O₂. Bevorzugt wird H₂O₂ langsam oder portionsweise dem Reaktionsgemisch aus Lösungsmittel, Substrat und Katalysator zugegeben, wobei das Reaktionsgemisch gerührt wird.
Der Verbrauch an H₂O₂ ist bei dem erfindungsgemäßen Verfahren vom eingesetzten Substrat abhängig. Für reaktive Substrate werden bevorzugt 2 bis 3 Äquivalente an H₂O₂ benötigt, während weniger reaktive Substrate bevorzugt mit 3 bis 10 Äquivalenten an H₂O₂ umgesetzt werden.

Die Reaktionstemperatur liegt zwischen 0 und 50°C, bevorzugt zwischen 15 und 35°C.

Der Reaktionsverlauf kann mittels UV-Spektroskopie oder mittels HPLC verfolgt werden.
Nach beendeter Reaktion, d.h. nach 1 bis 30 Stunden je nach Reaktionsbedingungen, erfolgt die Aufarbeitung des Reaktionsgemisches. Die Abtrennung des Katalysators gestaltet sich unerwarteterweise insbesondere bei Verwendung von Molybdat-Katalysatoren, wie z.B. von Natrium-Molybdat, in einigen Lösungsmitteln besonders einfach. Obwohl die Reaktion bei Verwendung von Molybdat-Katalysatoren, wie etwa Na₂MoO₄.2H₂O in mono-hydroxylischen, alkoholischen Lösungsmitteln, d.h. in einwertigen Alkoholen wie etwa Methanol oder Ethanol, vollständig homogen verläuft solange H₂O₂ zugegeben wird, fällt der Katalysator, nachdem H₂O₂ vollständig zugegeben wurde, aus dem Reaktionsgemisch aus, wodurch der Katalysator durch einfaches Zentrifugieren oder Filtrieren abgetrennt und recycliert werden kann.
Das verbleibende Endprodukt kann gegebenenfalls mittels Umkristallisieren, Extrahieren oder Destillation gereinigt werden.

Das erfindungsgemäße Verfahren ermöglicht die Oxidation einer Vielzahl von hydrophoben Verbindungen und ist besonders vorteilhaft bei der Oxidation von wasserunlöslichen Substraten, die mit bisher bekannten chemischen Methoden nicht mit hohe Ausbeute oxidierbar waren. Besonders geeignet ist das erfindungsgemäße Verfahren demnach zur Oxidation von ungesättigten organischen Verbindungen, wie Terpenen, beispielsweise α-Terpinen und Citronellol, aromatischen Polycyclen, Steroiden, Furanen, Cyclopentadienen, Phenolen u.s.w., und allgemein für alle Verbindungen die mit ¹O₂ reagieren.

Durch das erfindungsgemäße Verfahren werden die gewünschten Endprodukte in hohen Ausbeuten von bis zu 100% mit hoher Reinheit erhalten.
Das erfindungsgemäße Verfahren zeichnet sich dabei durch die einfache Prozessführung aus, die sich bestens für den industriellen Maßstab eignet, da sie in einfachen Mehrzweckanlagen und mit einfachen Aufarbeitungsschritten erfolgen kann und für ein breites Spektrum an Substraten angewendet werden kann.

### Beispiel 1:

Zu einer thermostatisierten Lösung (25°C) von 2 mmol eines organischen Substrates (α-Terpinen oder β-Citronellol) in 4ml eines organischen Lösungsmittels (LM) wurden
a) 0, 2ml einer wässrigen 2mol/l Na₂MoO₄-Lösung oder
b) 0,4mmol Na₂MoO₄.2aq.
zugegeben. Diesem Gemisch wurden 0,08ml H₂O₂ (50%) zugesetzt. Nachdem das Reaktionsgemisch sich wieder gelb verfärbte wurden zwei weitere 0,08ml Portionen H₂O₂ (50%) zugegeben. Der Reaktionsverlauf wurde bei α-Terpinen mittels UV-Spektroskopie (266nm) und bei β-Citronellol mittels HPLC (MeOH/H₂O 70/30) verfolgt.

### Beispiel 2:

Zu einer thermostatisierten Lösung (25°C) von 2 mmol eines organischen Substrates (α-Terpinen oder β-Citronellol) in 4ml eines organischen Lösungsmittels (LM) wurden
a) 0, 2ml einer wässrigen 2mol/l Na₂MoO₄-Lösung oder
b) 0,4mmol Na₂MoO₄.2aq.
zugegeben. Diesem Gemisch wurden 0,25ml H₂O₂ (50%) in einer Portion zugesetzt. Der Reaktionsverlauf wurde bei α-Terpinen mittels UV-Spektroskopie (266nm) und bei β-Citronellol mittels HPLC (MeOH/H₂O 70/30) verfolgt.

Die verwendeten LM und die Conversion von α-Terpinen in Ascaridol und β-Citronellol in ein 1/1 Gemisch der entsprechenden Hydroperoxide sind aus Tabelle 1 ersichtlich:

**Tabelle 1:**

| Beispiel | Substrat | Lösungsmittel | Kat. a) oder b) | Conversion |
|---|---|---|---|---|
| 1 | α-Terpinen | Methanol | a | 100% nach 2h |
| 1 | α-Terpinen | Methanol | b | >95% nach2h |
| 2 | α-Terpinen | Methanol | a | >95% nach2h |
| 1* | α-Terpinen | Ethanol | a | 90% nach 22h |
| 1 | α-Terpinen | Formamid | a | 70% nach 21 h |
| 1 | α-Terpinen | N-Me-formamid | a | 75% nach 3h |
| 1 | α-Terpinen | DMF | a | 64% nach 21 h |
| 1 | α-Terpinen | Sulfolan | a | 70% nach 21 h |
| 1 | β-Citronellol | Methanol | a | 80% nach 3h |
| 1 | β-Citronellol | Formamid | a | 95% nach 3h |

| | | | | |
|---|---|---|---|---|
| * 3.te Portion H₂O₂ betrug 0,09ml | | | | |

### Beispiel 3: Produktisolierung aus Reaktionsgemisch mit Methanol als LM

Nach der in Tabelle 1 angegebenen Zeit wurde der ausgefallene Katalysator mittels Zentrifugieren aus dem Reaktionsgemisch entfernt. Der Niederschlag wurde zweimal mit absolutem Ethanol gewaschen und die vereinigten Lösungsmittelchargen (Methanol und Ethanol) wurden abrotiert. Das verbleibende Oxidationsprodukt wurde für eine NMR-Analyse in CDCl₃ aufgelöst. Im Fall von α-Terpinen wurde durch die Analyse die Bildung einer fast quantitativen Menge von >95%ig reinem Ascaridol bestätigt. Bei β-Citronellol wurden etwa 80% Produkt erhalten, das gemäß NMR-Analyse aus einem 1/1 Gemisch der zwei korrespondierenden Hydroperoxide bestand.

### Beispiel 4:

Zu einer Lösung von 325 µl α-Terpinen und 48.5 mg Na₂MoO₄.2H₂O in 4ml Methanol wurden bei 35°C 45 µl H₂O₂ (50%) zugegeben. Diesem Gemisch wurden 5 weitere 45 µl - Portionen H₂O₂ (50%) zugesetzt, sobald sich das rot gefärbte Reaktionsgemisch wieder gelb verfärbte. Nach 1,5 Stunden wurde das Reaktionsgemische mittels HPLC analysiert. Die Analyse ergab eine quantitative Bildung von Ascaridol.

### Beispiel 5.

Zu einer Lösung von 365 µl Citronellol und 97 mg Na₂MoO₄.2H₂O in 4ml Ethylenglykol wurden bei 25°C 80 µl H₂O₂ (50%) zugegeben. Diesem Gemisch wurden nach 1, 2 und nach 19 Stunden 3 weitere 80 µl -Portionen H₂O₂ (50%) zugesetzt. Die HPLC-Analyse ergab eine 100%ige Conversion mit einer Ausbeute von sekundärem Hydroperoxid von 38% und einer Ausbeute von tertiärem Hydroperoxid von 62%.

## Patentansprüche

1. Verfahren zur Oxidation von organischen Substraten mittels ¹O₂, **dadurch gekennzeichnet, dass** hydrophobe, organische Substrate aus der Gruppe Olefine, die 1 bis 6 C=C-Doppelbindungen enthalten; C₆-C₃₀ Phenole, Polyalkylbenzole, Polyalkoxybenzolen; polycyclische Aromaten mit 2 bis 6 aromatischen Ringen; sowie C₄-C₂₀ Heterocyclen mit einem O-, N- oder S-Atom im Ring, die unsubstituiert sein können oder ein- oder mehrfach mit Halogenen, Cyanid, Carbonylgruppen, Hydroxylgruppen, C₁-C₂₀-Alkoxygruppen, C₁-C₂₀-Alkylgruppen, C₆-C₂₀-Arylgruppen, C₂-C₂₀-Alkenylgruppen, C₂-C₂₀-Alkinylgruppen, Carbonsäuregruppen, Estergruppen, Amidgruppen, Aminogruppen, Nitrogruppen, Silylgruppen, Silyloxygruppen, Sulfongruppen, Sulfoxidgruppen oder mit einem oder mehreren NR¹R²-Resten, in denen R₁ oder R₂ gleich oder verschieden sein können und H; C₁-C₂₀-Alkyl; Formyl; C₂-C₂₀-Acyl; C₇-C₂₀-Benzoyl bedeuten, wobei R¹ und R² auch gemeinsam einen Ring bilden können, substituiert sein können, in einem organischem Lösungsmittel aus der Gruppe C₁-C₈-Alkohole, Formamid, N-Methylformamid, Dimethylformamid, Sulfolan und Propylencarbonat in Gegenwart eines homogenen Katalysators basierend auf Molybdän, Wolfram, Scandium, Vanadium, Titan, Zirkon, Praseodym, Neodym, Samarium, Europium, Terbium, Dysprosium, Holmium, Erbium, Ytterbium oder Lutetium in Form von Oxiden, Oxokomplexen, Nitraten, Carboxylaten, Hydroxiden, Carbonaten, Chloriden, Fluoriden, Sulfaten oder Tetrafluorboraten mit 30-70%igem H₂O₂ versetzt werden, worauf anschließend an die katalytische Zersetzung von H₂O₂ zu Wasser und ¹O₂ die Oxidation zu den entsprechenden Oxidationsprodukten erfolgt.

2. Verfahren nach Anspruch 1, **dadurch gekennzeichnet, dass** als Lösungsmittel Methanol, Ethanol, Propanol, i-Propanol, Ethylenglykol, Propylenglykol, Formamid, N-Methylformamid oder Dimethylformamid verwendet werden.

3. Verfahren nach Anspruch 1, **dadurch gekennzeichnet, dass** in Abhängigkeit vom eingesetzten Substrat 2 bis 10 Äquivalente an H₂O₂ eingesetzt werden.

4. Verfahren nach Anspruch 1, **dadurch gekennzeichnet, dass** die Reaktionstemperatur zwischen 0 und 50°C liegt.

5. Verfahren nach Anspruch 1, **dadurch gekennzeichnet, dass** im Anschluss an die Umsetzung der hydrophoben, organischen Substrate, die mit ¹O₂ reagieren, in einem einwertigen C₁-C₈-Alkohol als Lösungsmittel in Gegenwart eines Molybdat-Katalysators mit 30-70%igem H₂O₂ zu den entsprechenden Oxidationsprodukten, die Abtrennung und Recyclierung des ausgefallenen Katalysators nach vollendeter Reaktion durch einfaches Zentrifugieren oder Abfiltrieren erfolgt.

## Claims

1. A process for the oxidation of organic substrates by means of ¹O₂, which comprises adding 30-70% strength H₂O₂ to hydrophobic organic substrates from the group of olefins which contain 1-6 C=C double bonds; C₆-C₃₀ phenols, polyalkylbenzenes, polyalkoxybenzenes; polycyclic aromatics with 2 to 6 aromatic rings; and C₄-C₂₀ heterocycles with an O, N or S atom in the ring, which may be unsubstituted or may be mono- or polysubstituted by halogens, cyanide, carbonyl groups, hydroxyl groups, C₁-C₂₀-alkoxy groups, C₁-C₂₀-alkyl groups, C₆-C₂₀-aryl groups, C₂-C₂₀-alkenyl groups, C₂-C₂₀-alkynyl groups, carboxylic acid groups, ester groups, amide groups, amino groups, nitro groups, silyl groups, silyloxy groups, sulfone groups, sulfoxide groups or by one or more NR¹R² radicals in which R₁ or R₂ may be identical or different and are H; C₁-C₂₀-alkyl; formyl; C₂-C₂₀-acyl; C₇-C₂₀-benzoyl, where R¹ and R² may also together form a ring, in an organic solvent from the group consisting of C₁-C₈-alcohols, formamide, N-methylformamide, dimethylformamide, sulfolane and propylene carbonate in the presence of a homogeneous catalyst based on molybdenum, tungsten, scandium, vanadium, titanium, zirconium, praseodymium, neodymium, samarium, europium, terbium, dysprosium, holmium, erbium, ytterbium or lutetium in the form of oxides, oxo complexes, nitrates, carboxylates, hydroxides, carbonates, chlorides, fluorides, sulfates or tetrafluoroborates, whereupon, following the catalytic decomposition of H₂O₂ to give water and ¹O₂, oxidation to give the corresponding oxidation products takes place.

2. The process as claimed in claim 1, wherein the solvent used is methanol, ethanol, propanol, isopropanol, ethylene glycol, propylene glycol, formamide, N-methylformamide or dimethylformamide.

3. The process as claimed in claim 1, wherein 2 to 10 equivalents of H₂O₂ are used depending on the substrate used.

4. The process as claimed in claim 1, wherein the reaction temperature is between 0 and 50°C.

5. The process as claimed in claim 1, wherein, following the reaction of the hydrophobic organic substrates which react with ¹O₂ in a monohydric C₁-C₈ alcohol as solvent in the presence of a molybdate catalyst with 30-70% strength H₂O₂ to give the corresponding oxidation products, the removal and recycling of the precipitated-out catalyst when the reaction is complete is carried out by simple centrifugation or filtration.

## Revendications

1. Procédé d'oxydation de substrats organiques à l'aide de ¹O₂, qui est **caractérisé en ce que** l'on ajoute au substrat organique hydrophobe, du groupe des oléfines, qui contiennent 1 à 6 doubles liaisons C=C ; des phénols en C₆-C₃₀, des polyalkylbenzènes, des polyalcoxybenzènes ; des aromatiques polycycliques ayant 2 à 6 cycles aromatiques ; ainsi que des hétérocycles en C₄-C₂₀ avec un atome O, N ou S dans le cycle, qui peuvent être non substitués ou substitués une ou plusieurs fois, avec des halogènes, un cyanure, des radicaux carbonyle, des radicaux hydroxyle, des radicaux alcoxy en C₁-C₂₀, des radicaux alkyle en C₁-C₂₀, des radicaux aryle en C₆-C₂₀, des radicaux alcényle en C₂-C₂₀, des radicaux alcynyle en C₂-C₂₀, des radicaux acide carboxylique, des radicaux ester, des radicaux amide, des radicaux amino, des radicaux nitro, des radicaux silyle, des radicaux siloxy, des radicaux sulfone, des radicaux sulfoxyde ou avec un ou plusieurs résidus NR¹R², dans lesquels R¹ ou R² peuvent être identiques ou différents et représentent H, des radicaux alkyle en C₁-C₂₀, formyle, des radicaux acyle en C₂-C₂₀, des radicaux benzoyle en C₇-C₂₀, où R¹ et R² peuvent également former un cycle, dans un solvant organique du groupe des alcools en C₁-C₈, du formamide, du N-méthylformamide, du diméthylformamide, du sulfolane et du carbonate de propylène, en présence d'un catalyseur homogène à base de molybdène, de tungstène, de scandium, de vanadium, de titane, de zirconium, de praséodyme, de néodyme, de samarium, d'europium, de terbium, de dysprosium, de holmium, d'erbium, d'ytterbium ou de lutétium, sous la forme d'oxydes, de complexes oxo, de nitrates, de carboxylates, d'hydroxydes, de carbonates, de chlorures, de fluorures, de sulfates ou de tétrafluoroborates, de l'H₂O₂ à 30-70%, où ensuite on réalise l'oxydation en les produits d'oxydation correspondants par la décomposition catalytique de l'H₂O₂ en eau et ¹O₂.

2. Procédé selon la revendication 1, **caractérisé en ce que** l'on utilise comme solvant, le méthanol, l'éthanol, le propanol, le i-propanol, l'éthylèneglycol, le propylèneglycol, le formamide, le N-méthylformamide ou le diméthylformamide.

3. Procédé selon la revendication 1, **caractérisé en ce que** l'on met en oeuvre 2 à 10 équivalents de H₂O₂ en fonction du substrat mis en oeuvre.

4. Procédé selon la revendication 1, **caractérisé en ce que** la température de réaction se situe entre 0 et 50°C.

5. Procédé selon la revendication 1, **caractérisé en ce que** à la fin de la. réaction du substrat hydrophobe organique, qui réagit avec ¹O₂, dans un alcool monovalent en C₁-C₈ comme solvant, en présence d'un catalyseur au molybdate avec H₂O₂ à 30-70%, en les produits d'oxydation correspondants, on réalise la séparation et le recyclage du catalyseur précipité lorsque la réaction est complète, par simple centrifugation ou filtration.
